# EUROPEAN PATENT SPECIFICATION

(11) **EP 2 072 055 B1**
(45) Date of publication and mention of the grant of the patent: **23.03.2016**
(21) Application number: 07150338.7
(22) Date of filing: 21.12.2007
(51) Int. Cl.: A61K 36/185, A61K 36/062, A61P 25/28

(54) **Composition and method for prevention and treatment of Alzheimer's disease**
Zusammensetzung und Verfahren zur Prävention und Behandlung von Alzheimer
Composition et procédé de prévention et traitement de la maladie d'Alzheimer

(43) Date of publication of application: 24.06.2009
(73) Proprietor: Sunway Biotech Co., Ltd., Taipei City 114, (TW)
(72) Inventor: Pan, Tzu-Ming, 221, Xizhi City, Taipei County (TW); Lee, Chun-Lin, 830, Fongshan City, Kaohsiung County (TW)
(74) Representative: Becker Kurig Straus

(56) References cited:
- US-A1- 2006 078 533
- LEE CHUN-LIN ET AL: "Red mold rice ameliorates impairment of memory and learning ability in intracerebroventricular amyloid beta-infused rat by repressing amyloid beta accumulation" November 2007 (2007-11), JOURNAL OF NEUROSCIENCE RESEARCH, VOL. 85, NR. 14, PAGE(S) 3171-3182 , XP009108515 ISSN: 0360-4012 * page 3171, right-hand column * * page 3180, right-hand column, last paragraph - page 3181, left-hand column, paragraph F *

## Description

### BACKGROUND OF THE INVENTION

### 1) FIELD OF THE INVENTION

This invention relates to a composition for use in the prevention and treatment of Alzheimer's disease, in particular, to a composition comprising monacolins, anti-inflammation and anti-oxidant agents which are extracted from red mold rice (RMR) and applied in various forms of pastils, capsules, powder, beverage, etc.

### 2) DESCRIPTION OF THE PRIOR ART

Alzheimer's disease (AD) is associated with a progressive neurons failure. AD is a major cause of dementia, most often dementia and AD are used interchangably. In Alzheimer's disease (AD), the progressive loss of cognitive, language and emotional functions occurs. Generally, serious AD patients need more care and require assistance in all respects, such as taking a bath, eating, going to toilet, etc. Therefore, there are great impacts on the families of those AD patients in their daily lives. Loss of memory is the most often seen symptom of AD. At the very beginning, the symptom may not be conscious by AD's family around and the aforementioned symptoms do not correlate with AD only. Some known symptoms that seriously affects a person's ability to carry out daily activities including no recognition of a place or direction, without memory of recent events, bringing up a matter of the past repeatedly or unable to learn new things. As the disease gets worse, people often have difficulty expressing him/herself or making decisions. Gradually there is no recognition of family and relatives for AD patients. Some AD patients may be in great agitation, paranoiac, suspecious, dislike interacting with people. Subsequently, AD patients may wander on the street or lose their way home. New studies show that the brain damages to AD patients involved in human vision and sense of space, so that an AD patient has the problem in identifying a clear direction or find the way. AD patients may have difficulty concentrating and can not carry out daily activities by themselves. Other damage to brain cells of AD patients, e.g., basal forebrain and hippocampus, may lead to memory loss and confusion. Many AD patients finally die of other causes rather than AD, such as pneumonia. Generally, AD patients can live 6-8 years, but many AD patients still survive for more than 20 years.

The Food and Drug Administration (FDA) of the U.S. has approved five pharmaceutical treatments for Alzheimer's disease in clinical use, including cholinesterase inhibitors (including tacrine and donepezil). Cholinesterase is an enzyme that catalyzes the hydrolysis of the neurotransmitter acetylcholine (AChE) into choline and acetic acid, currently, those approved pharmaceutical treatments are used to inhibit cholinesterase and repress the hydrolysis of the neurotransmitter acetylcholine (AChE). Both inhibitors are able to increase AChE content in the brain, defer the lose of memory, and allow AD patients to carry out their daily activities. Importantly, those pharmaceutical treatments are unable to cure Alzheimer's disease, but only relieve AD symptoms. Moreover, the aforementioned medications are proved to cause some side effects to AD patients, such as feel nauseated, headache, diarrhoea, insomnia, pain, illusion or dizzying, etc., therefore, the aforementioned medications are not practical Alzheimer's disease treatments.

Therefore, how to effectively prevent people from Alzheimer's disease and cure those AD patients without noticeable side effects caused need to be researched.

### SUMMARY OF THE INVENTION

In view of the foregoing drawbacks, the invention provides a composition for use in preventing and treating Alzheimer's disease according to claim 1.

Another objective of the invention is to provide a composition use in the prevention and treatment of Alzheimer's disease without noticeable side effects caused to AD patients.

The invention according to another aspects resides in a composition for use in the prevention and treatment of Alzheimer's disease, the composition comprising monacolins, anti-inflammation and anti-oxidant agents applied in various forms of pastils, capsules, powder, beverage, etc. in prevention and treatment of Alzheimer's disease. The composition of the invention, which is formed by the aforementioned three compounds, is used for the prevention and treatment of Alzheimer's disease. Moreover, in a preferred embodiment of the invention, the minimum effective dose of monacolins is at least greater than 100 ug, the minimum effective dose of anti-oxidant compounds is at least greater than 40 ug and the minimum effective dose of anti-inflammation agents is at least greater than 10 ug; wherein the monacolines, anti-inflammation agents and anti-oxidant compounds have the optimum weight in the ratio of 40:2:1, enabling the composition of the invention to achieve the objectives of prevention and treatment of Alzheimer's disease without causing noticeable side effects to AD patients.

A detailed description is given in the following embodiments with reference to the accompanying drawings.

### BRIEF DESCRIPTION OF THE DRAWINGS

Fig. 1 is a prospective view of an apparatus according to the invention;
Fig. 2 is a statistical chart showing the effect of RMR on step-through latency of multiple-trial passive avoidance task in the rats from a light chamber into a dark chamber;
Figs. 3-1 & 3-2 show perspective views regarding an apparatus of water maze according to the invention;
Figs. 4-A, 4-B & 4-C show influence diagrams regarding effects of RMR on performance of the memory and learning ability of the Aβ340-infused rats in the training trials of reference memory task and probe test;
Fig. 5 is a chart showing effect of RMR on performance of the memory and learning ability of the Aβ340-infused rats in the training trials of working memory task;
Fig. 6 is an influence diagram showing effect of RMR on activity of acetylcholinesterase (AChE) in the hippocampus and cortex of Aβ340-infused rats;
Fig. 7 is a schematic, diagram showing effect of RMR on the formation of total antioxidant status (TAS) activity in the hippocampus and cortex of Aβ340-infused rats;
Fig. 8 is an influence diagram showing effect of RMR on the formation of MDA activity in the hippocampus and cortex of Aβ340-infused rats;
Fig. 9 is an influence diagram showing effect of RMR on the formation of superoxide dismutase (SOD) activity in the hippocampus and cortex of Aβ340-infused rats;
Figs. 10-A & 10-B are diagrams showing effects of RMR on the formation of ROS in the hippocampus and cortex and iNOS expression of Aβ40-infused rats;
Fig. 11 is a diagram showing effect of RMR on the Aβ 40 accumulation in the hippocampus of Aβ40-infused rats; and
Figs. 12-1∼12-3 are flowcharts showing the method of making the composition of the invention for prevention and treatment of AD patients.

### DETAILED DESCRIPTION OF THE PREFERRED EMBODIMENTS

To enable a further understanding of the structural features and the technical contents of the invention, the brief description of the drawings is followed by the detailed description of embodiments.

The invention disclosed herein is a composition for use in the prevention and treatment of Alzheimer's disease without causing noticeable side effects. Prior to introduction to the composition of the invention, some concepts of the invention are described as follows. Agitation and accumulation of Amyloid β peptide (Aβ) is the pathogenic agent of Alzheimer's disease (AD), leads to neurotransmitter deficits and repress oxidizaton and inflammatory response in the brain resulting in the aggravation of the AD situation for AD patients. Aβ is formed after sequential cleavage of the amyloid precursor protein (APP) through α, β and γ-secretase.

The formation of Aβ is carried out by the proteolytic cleavage of 671 and 672 amino acid binding site perfomed by β-secretase, as well as the cleavage of 713 amino acid binding site of APP performed by γ-sceretase. The cleavage performed by secretase results in not only 1-40 and 1-42 fragment but also other fragment. APP would be cleaved in sAPP-α and p10 fragments by α-secretase. However, p10 would be further cleaved to p3, a part of Aβ, and p7 fragments. Furthermore, the cleavage of APP performed by β-secretase would result in the formation of sAPPβ and p12 fragments, and futher cause p12 fragment to be cleaved to Aβ and p7 fragments though γ-secretase (Evin et al. 2003; Shoji et al. 1992). APP is cleaved to various types of Aβ though β- and γ-secretase. However, soluble Aβ without neurotoxicity would be aggregated as Aβ fibrils with neurotoxicity which leads to neuron damage via the balance disorder of calcium ion and oxidative stress. Aj3 fibrils also break neurite outgrowth and cell death though promoting hyperphosphorylation of tau protein. In addition, Aβ fibrils would bind to the specific receptor on the membrane of microglia and astrocyte, which leads to the activation of microglia involved in the release of many neurotoxic factors such as proinflammatory factors: IL-6, IL-1, and TNF-α as well as NO, ROS, and free radical. Currently, Apolipoprotein E gene in the 19th pair of chromosome is found to associate with AD pathogenesis. ApoE is able to easily and rapidly bind to Aβ, and further result in the deposition of senile plaque involved in neurotoxicity.

In addition, many documents and literatures indicate that there is a certain relation between cardiovascular disease and Alzheimer's disease. Researches by Martha et al focused their experiments on elders above 65 years old and elucidated the results that those elders who ingested saturated fats during their diet easily have cardiovascular disease and most of those elders suffered from Alzheimer's disease 4 years on average after they were with diagnosed cardiovascular disease; in contrast, there was an inverse correlation if those elders whose diet contained abundant polyunsaturated fat and monounsaturated fat (Freund-Levi et al. 2006). Many researches have proved that there is a relation between Aβ production, which promotes Alzheimer's disease, and intracellular lipid metabolism (Frears et al. 1999; Kuo et al. 1998; Roher and Kuo 1999). Recent researches have found that Aβ alters due to intracellular cholesterol distribution and cholesterol esterification (Frears et al. 1999). ApoE increases plasma cholesterol levels and is one of the risk factors associate with cardiovascular disease. The experiment (Puglielli et al. 2001) showed that there is a positive correlation between acetylcholinesterase (AChE) activity and cholesterol ester (CE) content in the rat cells (Puglielli et al. 2001; Zhao et al. 2005). Therefore, it is found from researches that there is a positive correlation between cardiovascular disease and Alzheimer's disease.

Consequently, the composition of the invention is used to decrease the Aβ accumulation in the brain and reduce saturated fats which may contribute to cardiovascular disease, so as to remedy Alzheimer's disease.

In recent year, there are many researches associated with *Monascus* species. In the future, the development of RMR ingredients and compounds may become part of health food with multi-functions for human beings. The invention proves that monacolin K, γ-aminobutyric acid (GABA) and anti-oxidant compounds in RMR are substantially enhance the feasibility for prevention and treatment of Alzheimer's disease.

Monacolin K is lovastatin, a 3-hydroxy-3-methylglutaryl-coenzyme A (HMG CoA) reductase inhibitor (statin) for lowering cholesterol levels, and is clinically proved to effectively improve the symptoms of cardiovascular disease (CAD). In recent years, researches in epidemiology have found that statin has great clinical effectiveness for treatment of Alzheimer's disease. In UK General Practitioners Research Database that recent clinical reports describe an association between statin therapy and a reduction in the occurrence of Alzheimer's disease by as much as 70 % (Jick et al. 2000). Similar researches include an experiment that focused on AD patients above 60 years old in three hospitals in the USA, their AD diagnosis is based on the standard of National Institute of Neurological and Communicative Disorders and Stroke and the Alzheimer's Disease and Related Disorders Association (NINCDS-ADRDA). The experiment divided those AD patients into three groups, group A was a control group, group B was remedied by statins, group C was remedied by other cardiovascular disease medications (including medicine for lowering blood pressure), the experiment results showed surprisingly that the group of AD patients with statins remedy enabled the AD levels to decrease 70%, wherein the lovastatin and pravastatin had the best results for AD treatment (Wolozin et al. 2000).

Nerve impulse transmission agents, including nicotinic, muscarinic, serotonin, glutamate receptor and γ-aminobutyric acid (GABA), norepinephrine in the brain of AD patients are damaged. During aging process, human brain volumn will reduce 10%, mainly the quantity of cerebral cortex neuron, some brain area quantity will reduce 30-50% of volumn and neurotransmitters are also reduced, such as AChE, GABA, catecholamine, etc. In view of the foregoing, AD patients will gradually lose their brain cognition or memory ability. AD symptoms in early stage include memory deficit, confusion, or difficulty making decisions. In later AD period, patients have the symptoms of language disorder, abnormal behaviors, spasm, unable to carry out daily activities (Mohr et al. 1994).

When O₂ or H₂O₂ is deficient and OH · is excessive in mitochondria, OH · is formed with high response. Oxidative free radicals enable Aβ to form insolubile Aβ, and worsen AD symptoms (Hsieh and Tai 2003). It is found from experiment results that oral anti-oxidants such as a combination preparation comprising vitamin C and vitamin E is able to effectively alleviate AD symptoms (Yallampalli et al. 1998; Yamada et al. 1999). Ceramlde, a natural neurilemma, is used to against neuronal damage led by Aβ and FeSO₄ in hippocampus (Abousalham et al. 2002). In 1999, Aniya et al proposed the anti-oxidation ability of *Monascus* that anti-oxidant mechanism of dimerumic acid in *Monascus* is known to be able to eliminate the inhibition of LPO by ·OH, ·O²⁻, ferryl-Mb and peroxyl radicals; *Monascus* provides an electron for oxide to oxidize itself into nitroxide radical, and then nitroxide radical scavenging will lead to an anti-oxidant effect (Taira et al. 2002). Anti-oxidant effect from RMR is applicable to prevention of Alzheimer's disease, so that AD patients' condition can be improved without being aggravated due to free-radical induced oxidation.

Ths invention is published on 30 July 2007 on Journal of Neuroscience Research (Lee CL, Kuo TF, Wang JJ, Pan TM: Red mold rice ameliorates impairment of memory and learning ability in intracerebroventricular amyloid beta-infused rat via repressing amyloid beta accumulation. J Neurosci Res. 2007, 85, 3171-3182). In the experiment of the invention, male Wistar rats (weighing 250 g, 8 weeks old) were obtained from the Laboratory Animal Center of National Taiwan University College of Medicine. They were divided into 7 rats per group and kept in a temperature-controlled room (23±1□) under a 12-hr light : 12-hr dark cycle (light on at 08:00 and off at 20:00) and were given free access to food and water. When the weight is approximately 300 g, those rats were divided into groups with brain infusion. The daily RMR dietary and Aβ40 infustion are as shown in Table 1. The rats were divided to groups including 1) vehicle infused rats (vehicle group), surgery for i.c.v. with vehicle solution; 2) Aβ40 infused (Aβ group), which were infused i.c.v. with Aβ40 solution; 3) Aβ40 infused with administration of lovastatin (LS group), 4) Aβ40 infused with administration of a onefold dosage of RMR (RL group) and 5) Aβ40 infused with administration of a fivefold dosage of RMR (RH group). The dosage of RMR is calculated in accordance with Boyd's formula of body surface area as recommended by the Food and Drug Administration (Boyd 1935; Lee et al. 2006). 2 g of RMR was used as the onefold dosage for an adult with a weight of 65 kg and a height of 170 cm. RH group of rats were daily administered high dosage of RMR, LS and RL groups of rats were administered the same dosage of lovastatin (1.43 mg/kg/day, per rat) for comparing the effects between applying simply monacolin K and applying RMR composition, including comprising monacolins, anti-inflammation agents and anti-oxidant compounds, for the prevention and treatment of AD.

Rats were injected with Aβ40 infusion for 28 consective days in the brain to develop Alzheimer's disease. The daily dietary dose of RMR products or the same lovastatin contents was used for those rats to find out the RMR outcome against Aβ-induced neurotoxicity and evaluate effect of RMR on the memory and learning ability of the rats. The results of the experiment of the invention show that infusion of Aβ40 for 28 consective days in the brain increased the AChE activity, reactive oxygen species (ROS) content and lipid peroxide (LPO) levels, and at the same time reduce Total antioxidant capacity (T-AOC) and superoxide dismutase (SOD) activity. The dietary dose of RMR significantly repressed the Aβ40 infusion damage to the brain and had better effect compared with lovastatin group. Moreover, Aβ40 infusion was not able to considerably accumulate in hippocampus through repressing oxidizaton and inflammatory response. Red mold rice (RMR) is at the first time proved to have the effect on decreasing the risk factor for memory deficit and dementia in AD patients and the effects is significant compared with lovastatin group.

The animals grouping and experiment schedule of the invention are as follows :

**Table 1**

| Groups | Aβ Infusion | RMR (monacolin K) (mg/kg rat per day) | Lovastatin (mg/kg rat per day) |
|---|---|---|---|
| Vehicle | - | - | - |
| Aβ | + | - | - |
| LS | + | - | 1.43 |
| RL | + | 151 (1.44) | - |
| RH | + | 755 (7.20) | - |

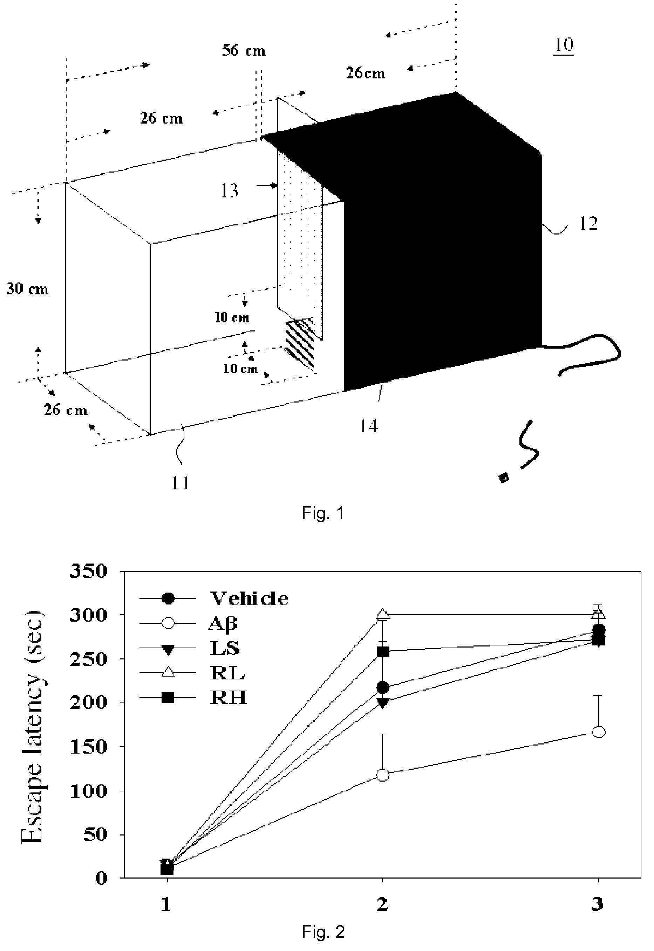

With reference to Fig. 1, an apparatus 10 consisted of a light chamber 11 and a dark chamber 12; the chambers were both the same size. A shuttle door 13 of 10 (W) × 10 (D) cm was set for separation of the two chambers. When the shuttle door 13 was opened, the light chamber 11 and the dark chamber 12 are open to each other. The light chamber 11 is with illumination, whereas the dark chamber 12 is not. A plurality of electric wires 14 arranged with a parallel interval of 1 cm were set through the floor of the dark chamber 12 and delivered an electric shock when the door was closed. In each trial, a rat was placed into the light chamber 11 first and the shuttle door 13 was opened. After the rat entered the dark chamber 12, the shuttle door 13 was immediately closed and the retention time was recorded, and an inescapable electric shock (100 V, 0.3 mA, 2 sec) was delivered through the electric wires 14, the rat was removed from the dark chamber after 5 sec. of foot shock. If the rat was indifferent to go into the dark chamber 12 after 300 sec., the rat was compelled to go into the dark chamber 12 and received electric shock through the electric wires 14 arranged on the floor of the dark chamber 12 after the shuttle door 13 was closed. Subsequently, the rat was again placed into the light chamber 11 with the shuttle door 13 opened, and the step-through latency of the rat in the light chamber 11 was measured in the retention test performed 24 hours and 48 hours after the training trial. If the time the rat stayed in the light chamber 11 was measured above 300 sec., memory and learning ability of the rat was considered normal without problem.

The step-through latency of a rat from the light chamber 11 to the dark chamber 12 was used as a marker to evaluate the memory and learning ability in the passive avoidance task. As shown in Fig. 2, all the rats would immediately go into the dark chamber 12 in the first trial because of skoto-taxis, but the electric shock in the dark chamber 12 should intimidate and prevent rats with normal memory ability from going into the dark chamber 12 the next time. Therefore, the step-through latency among each group of rats in the light chamber 11 would show the most significant difference in the second trial. The results of the second trial clearly indicate that Aβ-infused rats still spent shorter times staying in the light chamber than vehicle-infused rats. However, RL and RH groups are able to ensure staying in the light chamber for a longer time compared with the Aβ group (p<0.05). The effect on lovastatin group is lower than that on RL group with the same monacolin K contents, but the performance on the memory and learning ability improvement of lovastatin group is significantly higher than that ofAβ group.

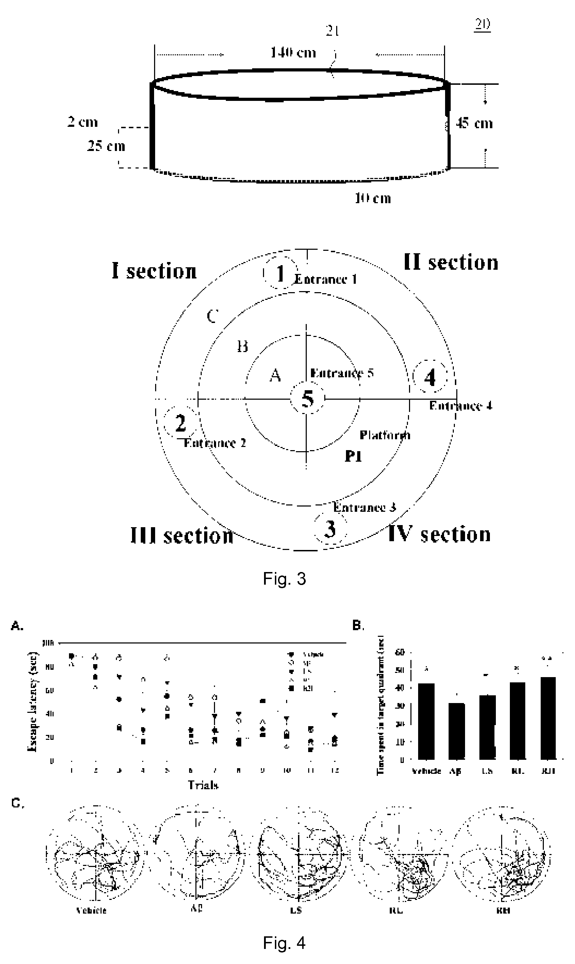

With reference to Fig. 3, the invention provides a water maze 20, comprising a circular tank 21 (diameter 140 cm, height 45 cm), which was used as an apparatus of the water maze 20 in which a movable escape platform P1 (diameter 12 cm, height 25 cm.) was located inside the tank 21. Prior to the experiment, the circular tank 21 was filled with water to a height of 27 cm. The circular tank 21 was divided into four quadrants (I, II, III and IV), there were five starting positions set in the tank 21 and a position with equal distance from center and edge in the middle of each quadrant was marked for the location of platform P1. During the experiment, a camera was set at the ceiling above the center of the water tank 21 for recording swimming routes of those rats.

Fig. 4 shows the influence effects of RMR on performance of the memory and learning ability of the Aβ40-infused rats. The time that rats started from a starting point of the apparatus of water maze 20 to search the escape platform P1 is regarded as the index for evaluation of the reference memory task. The experiment results show that the Aβ-infused group always has longer escape latency of finding the escape platform P1 from the second trial to the ninth trial compared with the vehicle group. The dietary administration of RMR (RL and RH groups), however, signigicantly decreases escape latency from the second trial to the ninth trial compared with the Aβ-infused group (p<0.05). Lovastatin administration also results in shorter escape latency compared with the Aβ group, but the effect would be weaker than in the RL and RH groups. Changes in path length produced by training trials in each group of rats showed a pattern similar to that of escape latency. There also were no significant differences in swiming speed among those groups of animal during the course of the training trials (data not shown) (p>0.05).

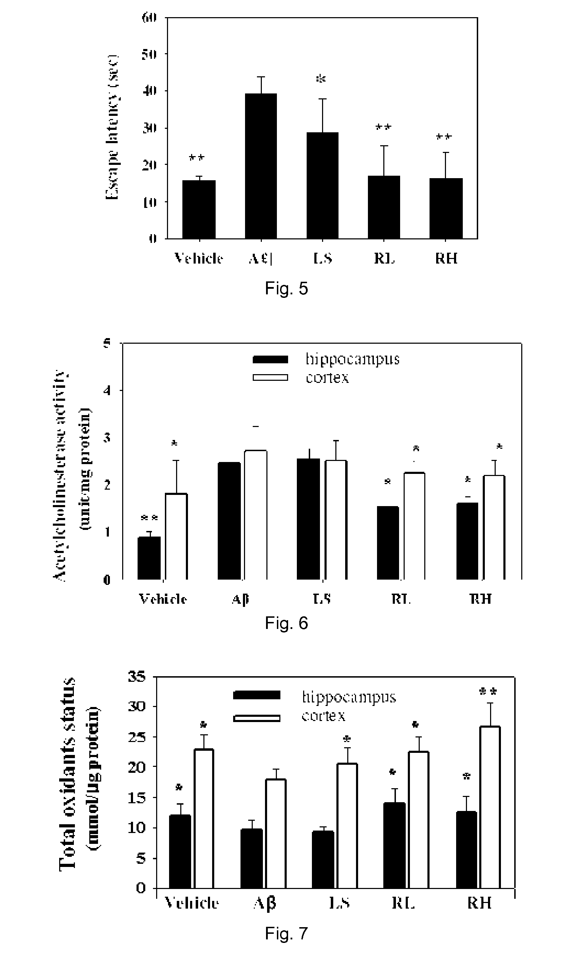

Probe test was immediately carried out after the training trial of the last reference memory task on day 24. The escape platform P1 was removed from the apparatus of water maze 20 (water tank 21). The time that rats wandered in the original quadrant with the escape platform P1 placed therein is regarded as the index of the probe test for evaluation of the memory and learning ability. The results showed in Fig. 4-A that the Aβ group spent less time searching the target quadrant than the vehicle group (p<0.05). Administration with RMR at onefold dosage in RL group or fivefold dosage in RH group results in significant increase on search time in the target quadrant by 38.2% (p<0.05) and 48.0% (p<0.01), respectively, compared with the As group, suggesting that the increase in search time in the RL and RH groups is attributable to the effects of RMR. It proves that red mold rice is able to improve the memory and learning ability for AD. Swiming pathway is helpful in understanding the truth of the memory and learning ability of rats in spatial probe trial. Fig. 4-B clearly indicated that Aβ-infused rats searched the target quadrant with directionless escape and around the whole circular tank 21. In contrast, rats with better memory and learning ability, such as RL, RH and vehicle groups, swam directly to the target quadrant and lingered for a long time. However, the lovastatin group always resulted in an ordinary ameliorative effect between RL group and Aβ group on impairment of memory and learning ability (Figs. 4-B and 4-C). The working memory task is a method for evaluation of short-term the memory and learning ability. With reference to Fig. 5, Aβ-infused rats are unable to shorten the escape latency time in searching the escape platform P1 compared with vehicle group (p<0.01). However, both RL and RH groups are able to perform memory and learning as fast as the vehicle group in the working memory task, and RL and RH groups significantly decrease escape latency time by 57.3% and 58.9% compared with the Aβ group (p<0.01). LS group significantly decreases escape latency time by 26.7% compared with the Aβ group (p<0.05), but the effect was weaker than with the RL and RH groups (p<0.05).

Aβ has been proved to be the risk factor for memory deficit and dementia in AD patients (Hashimoto et al. 2005; Stephan and Phillips 2005) and intracerebroventricular (i.c.v.) infusion of Aβ40 into lateral ventricle of rats has been demonstrated as a successful method for establishing an AD animal model (Stephan and Phillips 2005). Learning ability and memory deficit were tested in the behavior of Aβ-infused AD rats (Kar et al. 1998; Schubert et al. 1995; Townsend and Pratico 2005). The free radials and ROS induced by Aβ deposit damaged the neuron and synaptic junction and finally led to memory deficit and lack of and learning ability (Townsend and Pratico 2005). Therefore, antioxidants and anti-inflammation agents have usually been tried to reduce these AD risk factors in brain and ameliorate the impairment of memory and learning ability (Chauhan et al. 2004; Cordle et al. 2005).

Morris water maze task is used to evaluate the memory and learning ability, the reference memory task is a method of evaluating long-term memory ability and the working memory task is a method of evaluating short-term memory ability. According to the results of the aforementioned memory and learning tasks, the AD rats with onefold dosage and fivefold dosage of RMR shortened the escape latency time in the reference memory task and the working memory task (p<0.05). In addition, RMR dietary rats prolonged their searching time in the target wuadrant in probe trial. The experiment results clearly showed that rats with RMR dietary are able to improve the memory and learning ability to shorten the time in finding the escape platform in water maze. In contrast, AD rats without RMR dietary only searched the whole water maze with directionless escape and spent more time in the reference memory task and working memory task.

Monacolin K, an inhibitor of HMG-CoA reductase, was regarded as an important metabolite with hypolipidemic ability in RMR, statin are reported as a novel remedy for AD via the repression of Aβformation and Aβ-induced inflammatory response (Chauhan et al. 2004; Li et al. 2006). Many studies related to AD have used statins to lower A)3 formation in an AD transgenic mouse model (Yamada et al. 1999). Lovastatin has never been used to ameliorate the impairment of memory and learning ability in the Aβ-infused AD rat model. In this study, lovastatin was used to substitute for monacolin K of RMR in order to investigate whether ameliorating the impairment of memory ability by RMR administration resulted only from monacolin K. The results on the memory task clarified that lovastatin results in a weaker effect on ameliorating the impairment of memory than in the RL group even though the two groups included equal levels of monacolin K or lovastatin. Therefore, monacolin K is not the only functional ingredient to ameliorate Aβ-induced memory impairment.

Currently, the treatment and remedy of AD mostly focus on inhibition of AChE activity to increase AChE concentration and improve brain cognition and memory ability (Nabeshima and Nitta 1994). Relevant researches by establishing AD animal model also proved that the neurotransmitter AChE in the brain would be significantly decreased in the Aβ-infused rats. Some researches also proved that Aβ-infused rats have substantially less AChE contents than normal rats (Arendt et al. 1984; Darvesh et al. 2004). The decrease in AChE concentration and an increase in AChE activity caused serious neuron loss (Stephan and Phillips 2005). In addition, the increase in AChE activity was proved to stimulate Aβ aggregation in vitro and to form stable complexes with Aβ fibrils (Stephan and Phillips 2005). Therefore, inhibition of AChE activity is regarded as neuro-protection mechanism to indirectly lower Aβ-induced memory deficit. Fig. 6 is an influence diagram showing effect of RMR on activity of acetylcholinesterase in the hippocampus and cortex of Aβ40-infused rats. Aβ i.c.v. infusion leads to an increase in AChE activity in cortex by 50.5% and by 179.1% in hippocampus compared with the vehicle group. Administration with onefold or fivefold dosages of RMR significantly inhibits the Aβ-increased AChE activity, but lovastatin is ineffective at inhibiting AChE activity in cortex and hippocampus. The results suggest that the ingredient in RMR with an inhibitory effect on Aβ-raised AChE activity should be not monacolin K but the other functional metabolites. Not many researches elucidated that lovastatin is able to inhibit AChE activity, even some researches pointed out that lovastatin does not have significantly inhibition effect on AChE activity. This research result matches the invention tendency. In addition to monacolin K, metabolite with effectiveness of RMR may include other AChE inhibitors. However, the metabolite, GABA of RMR, is also a neurotransmitter and is useful to improve cognition and memory ability of AD patients.

Aβ has been demonstrated to cause oxidative stress damage in AD brain. Therefore, repressing Aβ-induced oxidative stress was regarded as an important goal in drug development for AD. In Fig. 7, Aβ infusion decreases the total antioxidants status (TAS) levels of cortex and hippocampus by 20.9% and 20.4%, respectively, compared with the vehicle group. Lovastatin administration is able to increase TAS levels by 13.9% in cortex compared with the Aβ group, but it is ineffective in hippocampus. However, significant increases by 24.6% and 46.2% in TAS levels in cortex and hippocampus were seen in the RL group. However, the experiment results showed that fivefold dosage RMR is able to increase TAS levels in cortex and hippocampus in other groups compared with the Aβ group.

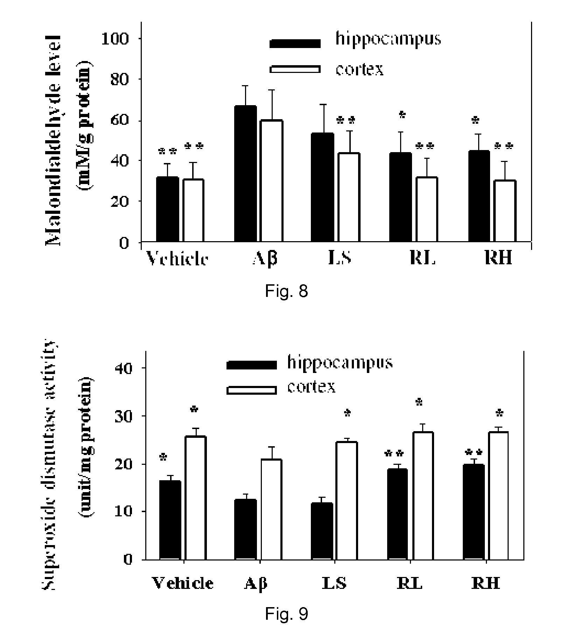

The effects of RMR administration on MDA levels of cortex and hippocampus are shown in Fig. 8. MDA levels are significantly increased by 95.3% and 112% in cortex and hippocampus, respectively, through Aβ infusion; but the MDA increase would be remarkably reversed with dose-response by increasing the dosage of RMR.

RL and RH groups also showed similar neuroprotective effects on superoxide dismutase (SOD) activity of cortex and hippocampus as shown in Fig. 9. Although SOD activity of cortex and hippocampus were reduced by Aβ infusion by 19.8% and 25.2%, respectively, RL and RH groups exhibited a significant increase in Aβ-reduced SOD activity by 27.2% and 52.7% in cortex and hippocampus with onefold dosage RMR and by 27.2% and 60.9% in cortex and hippocampus with fivefold dosage RMR.

In view of the foregoing, the rats in the experiment have serious oxidative stress damage in cortex and hippocampus. The oxidative stress damage can be improved by daily RMR dietary with dose-response and better effect than lovastatin group. Anti-oxidant compounds that are extracted from *Monascus*-fermented products include dimerumic acid, tannin, phenol, monounsaturated fatty acid and sterols (Aniya et al. 1999; Wang et al. 2006).

Therefore, the invention indicates that anti-oxidant compounds are able to be selected from either of the following compounds: dimerumic acid, tannin, phenol, monounsaturated fatty acid, sterols and superoxide dismutase (SOD).

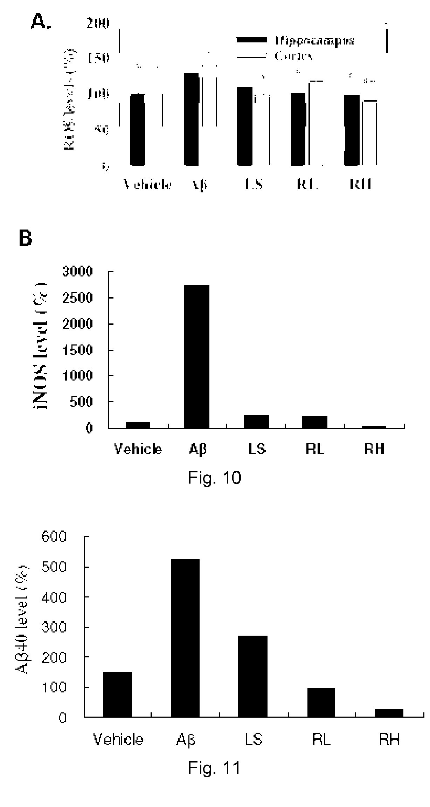

The experiment showed that the results of reactive oxygen species (ROS) levels in cortex and hippocampus as shown in Fig. 10-A. Aβ infusion significantly stimulates the increase of ROS levels in cortex and hippocampus by 39.8% and 28.7% (p<0.05). However, daily administration with RMR in RL and RH groups remarkably reduced Aβ-induced ROS levels in cortex and hippocampus by 16.0% (p<0.05) and 21.2% (p<0.05), respectively, in RL group and by 35.4% (p<0.01) and 21.3% (p<0.05), respectively, in RH group. The decrease in ROS levels by 29.3% in cortex and by 15.7% (p<0.05) in hippocampus were also found in the lovastatin group. The iNOS expression in hippocampus was shown in the immunohistochemical stain in Fig. 10-B. Aβ infusion resulted in a significant increase of iNOS expression, but the expression would be inhibited in the RL and RH groups. Importantly, iNOS expression of hippocampus in RL and RH groups is lower than that in the lovastatin group. The experiment results also showed that the anti-inflammation effect of lovastatin group is lower than that of RL and RH groups with dietary onefold and fivefold dosage RMR.

The significant decrease in ROS levels in cortex and hippocampus can be seen with dose response in the RMR administration groups. Although lovastatin has been used to lower Aβ-induced inflammation response in cell model, the neuroprotective effect of lovastatin has never been used to ameliorate the impairment of memory ability in Aβ-induced AD rat model according to our information. In this study, lovastatin administration also showed a significant decrease in ROS levels and iNOS expression compared with the Aβ group, but the effect was less than in the RL and RH groups. This is not the first report of the anti-inflammatory ability of RMR, the anti-inflammatory metabolites of RMR have been reported including various forms of monacolins; six azaphilones: monascin, ankaflavin, rubropunctatin, monascorburin, rubropunctamine and monascorburamine; two furanoisophthalides: xanthomonasin A and xanthomonasin B; and two amino acids: (+)-monascumic acid and (-)-monascumic acid (Schubert et al. 1995). It is clear that monacolin K is not the only functional metabolite repressing the Aβ-induced inflammation response. Another experiment (Akihisa et al 2005) showed that red mold rice repressed inflammation response induced by 12-O-tetradecanoylphorbol-13-acetate (TPA), the research also proved that major anti-inflammation agents are chemical compounds of azaphilones and furanoisophthalides. The results from this study and related researches regarding RMR against inflammation suggest that RMR repressing Aβ-induced inflammation response mainly results from the coordination mechanism collectively worked by monacolin K and other anti-inflammation agents.

Accordingly, the invention provides anti-inflammation agent anti-inflammation agent, which is able to be selected from the following compounds of γ-aminobutyric acid (GABA), monascin, ankaflavin, rubropunctatin, monascorburin, rubropunctamine, monascorburamine, xanthomonasin A, xanthomonasin B, (+)-monascumic acid and (-)-monascumic acid.

During the experiment, Aβ40 was continuously infused for 28 days into the brain in hippocampus of the rats. The results in Fig. 11 showed that Aβ40 accumulation in hippocampus of the Aβ group was higher than that in the vehicle group. It is known from the foregoing results and researches that Aβ340 infusion will cause oxidative stress and inflammation response in the brain, and progressively cause Aβ40 accumulation. More Aβ40 accumulation will cause more serious oxidative stress and inflammation response, so as to continuously aggravate brain damage in a vicious circle. Lovastatin has the effect of inhibiting Aβ40-induced inflammation response, but is weaker against oxidative stress. It is found from the experiment results that Aβ40 content of LS group in hippocampus was slightly lower than that of Aβ group, however, there was significant Aβ40 accumulation in the brain. The RL group and RH group with dietary dose of RMR had less Aβ40 accumulation in hippocampus than the Aβ group. The main reason why red mold rice is able to reduce Aβ40 accumulation in hippocampus lies in the ability of inhibiting oxidative stress and inflammation response. Aβ40-infused rats were not influenced by Aβ40 accumulation due to oxidative inflammation agents, so that Aβ40 was unable to cause damage to the brain so as to effectively improve the memory and learning ability.

Monacolins, anti-inflammation anti-oxidant compounds included in the composition for use in the prevention and treatment of Alzheimer's disease of the invention are extracted from red mold rice (RMR) to improve existing symptoms or slow Alzheimer's disease. In the first embodiment of the invention, the minimum effective dose of monacolins in 1 g of red mold rice is at least greater than 100 µg, the minimum effective dose of anti-oxidant compounds in 1 g of red mold rice is at least greater than 40 µg, and the minimum effective dose of anti-inflammation agents in 1 g of red mold rice is at least greater than 10 µg, wherein monacolins, anti-inflammation anti-oxidant compounds have the optimum weight in ratio of 40:2:1 as shown in the first embodiment to achieve the best effectiveness. In addition, monacolins, anti-inflammation anti-oxidant compounds have the optimum weights in ratio from 10:4:1 to 90:2:1 and the composition of the invention is able to be applied to in various forms of pastils, capsules, powder, beverage, etc.

Anti-inflammation agent in red mold rice (RMR) is γ-aminobutyric acid (GABA), red mold rice is regarded as natural food and is fermented and extracted through specific methods without causing side effects to human bodies. Therefore, RMR applications for the prevention and treatment of Alzheimer's disease only generate significant effectiveness without causing side effects on patients like general pharmaceuticals.

Thus, based on the experimental data presented abve, it is able to conclude that a composition for use in preventing and treating Alzheimer's disease consists of: monacolins, anti-inflammation and anti-oxidant agents wherein the said monacolins, anti-inflammation and anti-oxidant agents are extracted from a red mold rice, wherein the red mold rice is fabricated through a controlled fermentation process by using *Orvza sativa L.,* Japomica as a raw material rice of the red mold rice. Moreover, according to above descriptions, it can also know that the anti-inflammation agent is selected from the group consisting of a γ-aminobutyric acid (GABA), a monascin, an ankaflavin, a rubropunctatin, and a monascorburin; in addition, the anti-oxidant agent is selected from the group consisting of a dimerumic acid, a monounsaturated fatty acid and sterols. The most important teachnology feature for the composition is that, the monacolins, the anti-inflammation agents, and the anti-oxidant agents extracted from 1 g of the red mold rice have a weight percentage, and the weight percentage is 40:2:1.

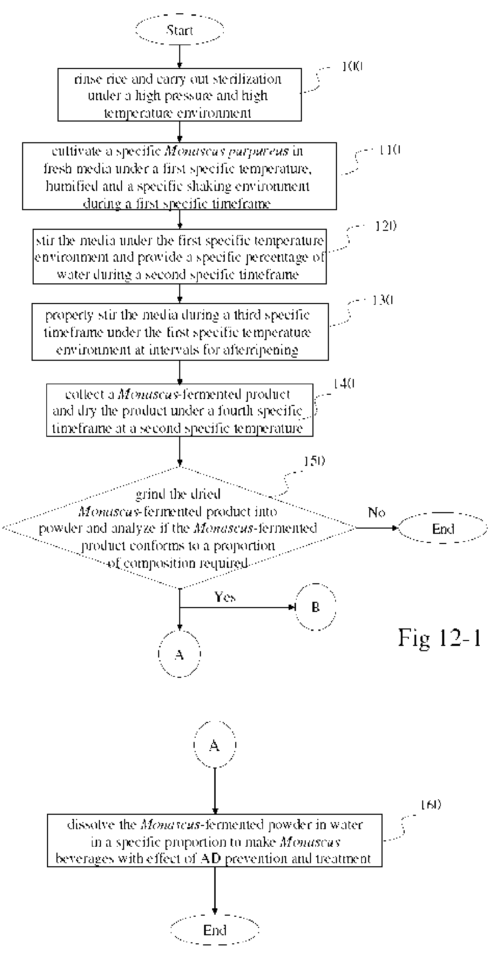

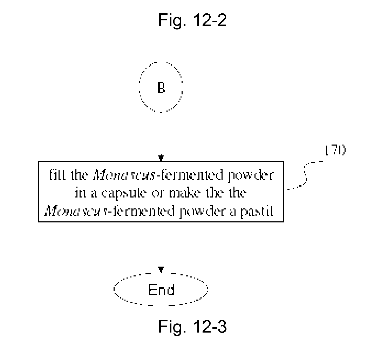

With reference to Figs. 12-1 ∼ 12-3, the invention provides a method for use in the prevention and treatment of Alzheimer's disease, the method comprises the following steps:
First of all, the step is to rinse rice and carry out sterilization under a high pressure and high temperature environment; the rice is long-grain rice (*Oryza sativa L.,* Japonica) purchased from a local supermarket in Taiwan to be used for RMR production under solid-state cultivation and the aforementioned high pressure and high temperature environment means under the environment at 121□ by the pressure of 1 kg/cm². The second step is to cultivate a specific *Monascus purpureus* in fresh media under a first specific temperature, humified and a specific shaking environment during a first specific timeframe, wherein the *_{M}onascus purpureus* in fresh media means at least 5 g of the rice is soaked in 100 mL. of sterile distilled water, the first specific timeframe is after 48 hours and the first specific temperature is maintained at 30 °C, the specific shaking environment is maintained at a rotational speed between 100 and 150 revolutions per minute (rpm); in other words, the *Monascus purpureus* is cultivated in the media and maintained in an environment at a temperature of 30 °C, a rotational speed 125 rpm and after 48 hours the cultivation is completed for collection. 500 g of solid-state cultivation is then collected, the *Monascus* is immersed in water for 6∼8 hours, filtered by cotton cloth and placed on a cloth in a plate for sterilization (121 °C, 20∼25 min). Another sterilization (121 °C, 20 min) is then carried out after 100 mL of water sprinkling. After cooling, another *Monascus* cultivation is carried out by placing *Monascus* in solid-state media (5%). Subsequently, the step is to sufficiently stir the media under the first specific temperature environment and provide a specific percentage of water during a second specific timeframe,
   wherein the second specific timeframe means within 72 hours, the specific percentage of water means 20% of sterile distilled water is supplemented; in other words, the *Monascus* is cultivated in a thermostated container at 30°C by way of stirring the media every 24 hours within 72 hours and supplementing with 20% of sterile distilled water. The next step is to properly stir the media during a third specific timeframe under the first specific temperature environment at intervals for afterripening,
   wherein the afterripening is the formation stage of metabolites and the third specific timeframe is within 96 hours. The fixed intervals are equal to every 10 hours. That is, the *Monascus* media is properly stirred every 10 hours within a total of 96 hours. Subsequently, after fermentation, the step is to collect a *Monascus-fermented* product and dry the product under a fourth specific timeframe at a second specific temperature,
   wherein the fourth specific timeframe means within 24 hours and the second specific temperature is maintained at the temperature range of 55-60 °C . This step is to collect *Monascus* and carry out the procedure of drying the *Monascus-*fermentedproduct for 24 hours at 60°C. The next step is to grind the dried *Monascus-* fermented product into powder and analyze if the *Monascus*-fermented product conforms to a proportion of composition required wherein the proportion of composition required means the composition comprising monacolins, anti-inflammation and anti-oxidant compounds at the effective weight ratios ranged from 10:4:1 to 90:2:1. If the *Monascus-fermented* powder does not conform to the proportion of composition required, the process is ended by failure. When the *Monascus-*fermented powder conforms to the proportion of composition required in the invention for prevention and treatment of AD, there are step A and step B to follow as shown in Figs. 12-2 and 12-3. Following step A, the final step is to dissolve the *Monascus*-fermented powder in water in a specific proportion to make *Monascus* beverages with effect on AD prevention and treatment and the process is completed. The specific proportion is between 1.0% and 4.0%.

Following step B, the final step is to fill the *Monascus-*fermented powder in a capsule or make the the *Monascus-*fermented powder a pastil.

The experiment for the invention is carried out through conventional media for *Monascus* production under solid-state cultivation, the koji dish size is 20 (L) _{×} 30 (W) × 5 cm (H) and a cloth is placed on the bottom of the media for easily stirring and maintaining the humidity, there is another cloth on the top for separation of external contamination and maintaining the moisture of *Monascus* during fermentation. The cultivation is carried out in an opening space. Consequently, the composition of the invention through the method proposed herein is able to be applied to in various forms of pastils, capsules, powder, beverage, etc.

## Claims

1. A composition for use in preventing and treating Alzheimer's disease, comprising:
monacolins, being extracted from a red mold rice, wherein the red mold rice is fabricated through a controlled fermentation process by using *Orvza sativa L.,* Japomica as a raw material rice of the red mold rice;
anti-inflammation agent being extracted from the red mold rice, and selected from the group consisting of a γ-aminobutyric acid (GABA), a monascin, an ankaflavin, a rubropunctatin, and a monascorburin; and
anti-oxidant agent being extracted from the red mold rice, and selected from the group consisting of a dimerumic acid, a monounsaturated fatty acid and sterols ;
wherein the monacolins, the anti-inflammation agent and the anti-oxidant agent extracted from 1 g of the red mold rice have a weight percentage, and the weight percentage is 40:2:1;
wherein the controlled fermentation process comprising a plurality of steps of:
(1) rinsing the raw material rice and carry out a first sterilization under 1 kg/cm² and 121 °C, and then the raw material rice of *Orvza sativa L*., Japomica is used as a cultivating media;
(2) soaking the cultivating media by a sterile distilled water;
(3) carrying out a first *Monascus* cultivation by cultivating a *Monascus purpureus* in the cultivating media under a first specific temperature of 30°C and a specific shaking environment of 100∼150 rpm/min during a first specific timeframe, so as to obtain a solid-state cultivation; wherein the first specific timeframe is after 48 hours;
(4) collecting the solid-state cultivation, and then immersing the solid-state cultivation in water for 6∼8 hours;
(5) filtering the solid-state cultivation by a cotton cloth for removing water, and then placing the solid-state cultivation on the cotton cloth in a plate for carrying out a second sterilization under 121 °C for 20∼25 min;
(6) after cooling, a second *Monascus* cultivation is carried out by placing a *Monascus purpureus* suspension (v/v, 5%) in the solid-state cultivation;
(7) sufficiently stirring the solid-state cultivation obtained from the step (6) media under 121 °C, and providing a specific percentage of water during a second specific timeframe, wherein the specific percentage of water means 20% of sterile distilled water is supplemented and the second specific timeframe, is within 72 hours;
(8) properly stirring the solid-state cultivation with the 20% sterile distilled water obtained from the step (7) every 10 hours within a total of 96 hours and under 121 °C, so as to form the red mold rice ;
(9) collecting the red mold rice;
(10) drying the red mold rice under 55-60 °C within 24 hours;
(11) grinding the dried red mold rice into a powdered red mold rice;
(12) determining whether the monacolins, the anti-inflammation agent, and the anti-oxidant agent extracted from 1 g of the powdered red mold rice have the weight percentage of 40:2:1, if yes, proceeding to step (13); and
(13) taking the powdered red mold rice as the composition for use in preventing and treating Alzheimer's disease;

2. The composition for use in preventing and treating Alzheimer's disease according to claim 1, wherein an adult dosage of the composition is 2 g/day.

3. The composition for use in preventing and treating Alzheimer's disease according to claim 1, wherein the composition is able to reduce the activity of an Acetylcholinesterase in Hippocampus and Cortex to at least 40% and 17.86% by ratio.

4. The composition for use in preventing and treating Alzheimer's disease according to claim 1, wherein the composition is able to reduce the level of an Malondialdehyde (MDA) in Hippocampus and Cortex to at least 30.77% and 41.67% by ratio.

5. The composition for use in preventing and treating Alzheimer's disease according to claim 1, wherein the composition is able to reduce the concentration of an Reactive Oxygen Species (ROS) in Hippocampus and Cortex to at least 21.2% and 16.0% by ratio.

6. The composition for use in preventing and treating Alzheimer's disease according to claim 1, wherein the composition is able to reduce the accumulation of a Beta-Amyloid 40 (Aβ340) and an inducible nitric oxide synthase (iNOS) in Hippocampus to at least 74.846% and 90.9% by ratio, respectively.

7. The composition for use in preventing and treating Alzheimer's disease according to claim 1, wherein the composition is able to increase the activity of a Total Antioxidant Status (TAS) in Hippocampus and Cortex to at least 46.2% and 24.6% by ratio.

8. The composition for use in preventing and treating Alzheimer's disease according to claim 1, wherein the composition is able to increase the activity of a Superoxide Dismutase (SOD) in Hippocampus and Cortex to at least 52.7% and 27.2% by ratio.

## Patentansprüche

1. Zusammensetzung zur Verwendung zur Vorbeugung und Behandlung der Alzheimer-Krankheit, umfassend:
Monacoline, extrahiert von Rot-fermentiertem Reis, wobei der Rot-fermentierte Reis durch einen gesteuerten Fermentierungsvorgang hergestellt wird, wobei *Orvza sativa L. Japonica* als Ausgangsmaterial-Reis für den Rot-fermentierten Reis verwendet wird,
ein entzündungshemmender Wirkstoff, extrahiert von dem Rot-fermentierten Reis und ausgewählt aus der Gruppe bestehend aus einer γ-Aminobuttersäure (GABA), einem Monascin, einem Ankaflawin, einem Rubropunctatin und einem Monascorburin, und
einen antioxidativen Wirkstoff, extrahiert aus dem Rot-fermentierten Reis, und ausgewählt aus der Gruppe bestehend aus Dimerumic-Säure, einer einfach ungesättigten Fettsäure und Sterinen,
wobei die Monacoline, der entzündungshemmende Wirkstoffe und der antioxidative Wirkstoff aus 1g des Rot-fermentierten Reises ein Gewichtsprozentverhältnis aufweisen, wobei das Gewichtsprozentverhältnis 40:2:1 beträgt,
wobei der gesteuerte Fermentierungsvorgang mehrere Schritte umfasst:
(1) Spülen des Ausgangsmaterial-Reises, und Ausführen einer ersten Sterilisation bei 1 kg/cm² und 121 °C, und dann Verwenden des Ausgangsmaterial-Reises *Orvza sativa L. Japonica* als Kultivierungsmedium,
(2) Einweichen des Kultivierungsmediums mit einem sterilen destilliertem Wasser,
(3) Durchführen einer ersten Kultivierung von Monascus durch Kultivieren von Monascus purpureus in dem Kultivierungsmedium bei einer ersten spezifischen Temperatur von 30 °C und einem spezifischen geschüttelten Milieu von 100∼150 /min während eines ersten spezifischen Zeitraums, um eine Kultivierung in einem festen Zustand zu erhalten, wobei der erste spezifische Zeitraum nach 48 Stunden vorliegt,
(4) Sammeln der Kultivierung im festen Zustand, und Tränken der Kultivierung im festen Zustand in Wasser für 6∼8 Stunden,
(5) Filtern der Kultivierung im festen Zustand durch ein Baumwolltuch, um Wasser zu entfernen, und danach anordnen der Kultivierung im festen Zustand auf dem Baumwolltuch in einer Tafel, um eine zweite Sterilisation bei 121 °C für 20∼25 Minuten durchzuführen,
(6) nach einem Abkühlen, wird eine zweite Monascus-Kultivierung ausgeführt in dem eine Monascus purpureus Lösung (v/v, 5%) in der Kultivierung im festen Zustand angeordnet wird,
(7) ausreichendes Rühren der Kultivierung im festen Zustand, die in dem Schritt (6) erhalten wurde bei 121 °C, und Bereitstellen eines spezifischen Prozentverhältnisses an Wasser während eines zweiten spezifischen Zeitraums, wobei das spezifische Prozentverhältnis Wasser eine Zugabe von 20% sterilen destilliertem Wasser bedeutet und der zweite spezifische Zeitraum innerhalb von 72 Stunden liegt,
(8) angemessen Rühren der Kultivierung im festen Zustand mit dem 20% sterilen destilliertem Wasser, das in dem Schritt (7) erhalten wurde, alle 10 Stunden, innerhalb von insgesamt 96 Stunden und bei 121 °C, um den Rot-fermentierten Reis zu bilden,
(9) Sammeln des rot fermentierten Reises,
(10) Trocknen des Rot-fermentierten Reises bei 55∼60 °C innerhalb von 24 Stunden,
(11) Mahlen des rot fermentierten Reises in einen pulverisierten Rot-fermentierten Reis,
(12) Bestimmen, ob die Monacoline, der entzündungshemmende Wirkstoff und der antioxidative Wirkstoff, die von 1 g des pulverisierten Rot-fermentierten Reis extrahiert wurden, ein Gewichtsprozentverhältnis von 40:2:1 aufweisen, und falls dies der Fall ist, fortfahren mit Schritt (13); und
(13) verwenden des pulverisierten Rot-fermentierten Reis als die Zusammensetzung zur Verwendung zur Vorbeugung und Behandlung der Alzheimer-Krankheit.

2. Zusammensetzung zur Verwendung zur Vorbeugung und Behandlung der Alzheimer-Krankheit nach Anspruch 1, wobei eine Dosis der Zusammensetzung für Erwachsene 2 g/Tag beträgt.

3. Zusammensetzung zur Verwendung zur Vorbeugung und Behandlung der Alzheimer-Krankheit nach Anspruch 1, wobei die Zusammensetzung in der Lage ist, die Aktivität einer Acetylcholinesterase im Hippocampus und der Hirnrinde im Verhältnis mindestens um 40% und 17,86% zu verringern.

4. Zusammensetzung zur Verwendung zur Vorbeugung und Behandlung der Alzheimer-Krankheit nach Anspruch 1, wobei die Zusammensetzung in der Lage ist, das Niveau eines Malondialdehyd (MDA) im Hippocampus und der Hirnrinde im Verhältnis mindestens um 30,77% und 41,67% zu verringern.

5. Zusammensetzung zur Verwendung zur Vorbeugung und Behandlung der Alzheimer-Krankheit nach Anspruch 1, wobei die Zusammensetzung in der Lage ist, die Konzentration einer reaktiven Sauerstoffspezies (ROS) im Hippocampus und der Hirnrinde im Verhältnis mindestens um 21,2% und 16,0% zu verringern.

6. Zusammensetzung zur Verwendung zur Vorbeugung und Behandlung der Alzheimer-Krankheit nach Anspruch 1, wobei die Zusammensetzung in der Lage ist, die Anreicherung eines Beta-Amyloids 40 (Aβ40) und einer induzierbaren Stickstoffoxid-Synthase (iNOS) im Hippocampus im Verhältnis mindestens um 74,846% bzw. 90,9% zu verringern.

7. Zusammensetzung zur Verwendung zur Vorbeugung und Behandlung der Alzheimer-Krankheit nach Anspruch 1, wobei die Zusammensetzung in der Lage ist, die Aktivität eines Gesamt-Antioxidans-Status (TAS) im Hippocampus und der Hirnrinde im Verhältnis mindestens um 46,2% und 24,6% zu erhöhen.

8. Zusammensetzung zur Verwendung zur Vorbeugung und Behandlung der Alzheimer-Krankheit nach Anspruch 1, wobei die Zusammensetzung in der Lage ist, die Aktivität einer Superoxid-Dismutase (SOD) im Hippocampus und der Hirnrinde im Verhältnis mindestens um 52,7% und 27,2% zu erhöhen.

## Revendications

1. Composition pour son utilisation dans la prévention et le traitement de la maladie d'Alzheimer, comprenant :
des monacolines, étant extraites d'une levure de riz rouge, dans laquelle la levure de riz rouge est fabriquée par un processus de fermentation régulé en utilisant *Oryza sativa L. Japonica* en tant que riz constituant la matière première de la levure de riz rouge ;
un agent anti-inflammatoire étant extrait de la levure de riz rouge, et sélectionné dans le groupe constitué d'un acide γ-aminobutyrique (GABA), d'une monascine, d'une ankaflavine, d'une rubropunctatine, et d'une monascorburine ; et
un agent antioxydant étant extrait de la levure de riz rouge, et sélectionné dans le groupe constitué d'un acide dimérumique, d'un acide gras mono-insaturé et de stérols ;
dans laquelle les monacolines, l'agent inflammatoire et l'agent antioxydant extraits de 1 g de la levure de riz rouge ont un pourcentage en poids, et le pourcentage en poids est de 40/2/1 ;
dans laquelle le processus de fermentation régulé comprend une pluralité d'étapes consistant à :
(1) rincer le riz constituant la matière première et réaliser une première stérilisation à 1 kg/cm² et 121°C, puis utiliser le riz constituant la matière première de *Oryza sativa L. Japonica* en tant que milieu de culture ;
(2) imprégner le milieu de culture d'eau distillée stérile ;
(3) réaliser une première culture de *Monascus* par culture d'un *Monascus purpureus* dans le milieu de culture à une température spécifique de 30°C et dans un environnement d'agitation spécifique de 100 à 150 tr/minute durant une première période spécifique, pour obtenir une culture à l'état solide ; dans laquelle la première période spécifique est après 48 heures ;
(4) collecter la culture à l'état solide, puis immerger la culture à l'état solide dans l'eau pendant 6 à 8 heures ;
(5) filtrer la culture à l'état solide avec un tissu de coton pour retirer l'eau, puis placer la culture à l'état solide sur le tissu de coton dans une plaque pour réaliser une seconde stérilisation à 121°C pendant 20 à 25 minutes ;
(6) après le refroidissement, réaliser une seconde culture de *Monascus* en plaçant une suspension de *Monascus purpureus* (v/v, 5 %) dans la culture à l'état solide ;
(7) suffisamment agiter la culture à l'état solide obtenue du milieu de l'étape (6) à 121°C, et fournir un pourcentage spécifique d'eau durant une seconde période spécifique, dans laquelle le pourcentage spécifique d'eau signifie que 20 % d'eau distillée stérile sont ajoutés et la seconde période spécifique dure 72 heures ;
(8) agiter correctement la culture à l'état solide avec les 20 % d'eau distillée stérile obtenue de l'étape (7) toutes les 10 heures pendant un total de 96 heures à 121°C pour former la levure de riz rouge ; i
(9) collecter la levure de riz rouge ;
(10) sécher la levure de riz rouge à 55 à 60°C pendant 24 heures ;
(11) broyer la levure de riz rouge en une poudre de levure de riz rouge ;
(12) déterminer si les monacolines, l'agent anti-inflammatoire et l'agent antioxydant extraits de 1 g de la poudre de levure de riz rouge ont le pourcentage en poids de 40/2/1 et, le cas échéant, procéder à l'étape (13) ; et
(13) prendre la poudre de levure de riz rouge comme composition à utiliser dans la prévention et le traitement de la maladie d'Alzheimer.

2. Composition pour son utilisation dans la prévention et le traitement de la maladie d'Alzheimer selon la revendication 1, dans laquelle une dose adulte de la composition est de 2 g/jour.

3. Composition pour son utilisation dans la prévention et le traitement de la maladie d'Alzheimer selon la revendication 1, dans laquelle la composition est capable de réduire l'activité d'une acétylcholinestérase dans l'hippocampe et le cortex d'au moins 40 % et 17,86 %.

4. Composition pour son utilisation dans la prévention et le traitement de la maladie d'Alzheimer selon la revendication 1, dans laquelle la composition est capable de réduire le niveau d'un malondialdéhyde (MDA) dans l'hippocampe et le cortex d'au moins 30,77 % et 41,67 %.

5. Composition pour son utilisation dans la prévention et le traitement de la maladie d'Alzheimer selon la revendication 1, dans laquelle la composition est capable de réduire la concentration d'une espèce réactive de l'oxygène (ROS) dans l'hippocampe et le cortex d'au moins 21,2 % et 16,0 %.

6. Composition pour son utilisation dans la prévention et le traitement de la maladie d'Alzheimer selon la revendication 1, dans laquelle la composition est capable de réduire l'accumulation d'une bêta-amyloïde 40 (Aβ40) et d'une oxyde nitrique synthase inductible (iNOS) dans l'hippocampe d'au moins 74,846 % et 90,9 %, respectivement.

7. Composition pour son utilisation dans la prévention et le traitement de la maladie d'Alzheimer selon la revendication 1, dans laquelle la composition est capable d'augmenter l'activité d'un statut antioxydant total (TAS) dans l'hippocampe et le cortex d'au moins 46,2 % et 24,6 %.

8. Composition pour son utilisation dans la prévention et le traitement de la maladie d'Alzheimer selon la revendication 1, dans laquelle la composition est capable d'augmenter l'activité d'une superoxyde dismutase (SOD) dans l'hippocampe et le cortex d'au moins 52,7 % et 27,2 %.
